Europäisches Patentamt

European Patent Office

Office européen des brevets

(11) Veröffentlichungsnummer: **0 229 907**
**A1**

## (12) EUROPÄISCHE PATENTANMELDUNG

(21) Anmeldenummer: 86115488.8

(22) Anmeldetag: 07.11.86

(51) Int. Cl.⁴: **A 61 F 13/20**

(30) Priorität: 18.12.85 DE 3544709

(43) Veröffentlichungstag der Anmeldung: 29.07.87
Patentblatt 87/31

(84) Benannte Vertragsstaaten: AT BE CH FR GB IT LI NL SE

(71) Anmelder: Vereinigte Papierwerke AG,
Schoppershofstrasse 80, D-8500 Nürnberg (DE)

(72) Erfinder: Reinheimer, Horst, Dr., Imkerweg 21,
D-8501 Heroldsberg (DE)
Erfinder: Männlein, Elisabeth, Dr., Franzosenweg 24,
D-8520 Erlangen (DE)
Erfinder: Stary, Christof, Am Steinbruch 11,
D-8501 Eckental (DE)

(74) Vertreter: Pohl, Hans Ludwig, Hefnersplatz 3,
D-8500 Nürnberg 11 (DE)

(54) Anal-Tampon.

(57) Es wird ein Anal-Tampon beschrieben, der aus einem Saugkörper aux Textilwatte oder dergl. besteht und der von einer flüssigkeitsdurchlässigen Hülle umgeben ist. Im Innern des Saugkörpers (2) ist ein Luftkissen angeordnet, welches aus einem allseits geschlossenen mit Luft oder dergl. gefüllten Folienschlauch besteht. Das Luftkissen kann auch in mehrere Kammern unterteilt sein.

EP 0 229 907 A1

86115488.8

0229907

Vereinigte Papierwerke          Schoppershofstr. 80
Schickedanz & Co.               8500 Nürnberg

                                HP/1 - B 73 D   12.Dez. 1985

## Anal-Tampon

Die Erfindung betrifft einen Anal-Tampon, der aus einem
Saugkörper aus Textilwatte, Zellstoff-Flocken oder dergl.
besteht und der von wenigstens einer flüssigkeitsdurchlässigen Hülle, beispielsweise einer solchen aus Vliesstoff umgeben ist.

Anal-Tampons dieser Art sind grundsätzlich bekannt. Sie
sind beispielsweise in der deutschen Offenlegungsschrift
14 91 151 beschrieben. Sie dienen als Schutz gegen unerwünschte Sekretion bei Anal-inkontinenten Patienten, aber
auch bei der Behandlung von Gefäßschwächen, die im Anal-
Bereich auftreten können, also beispielsweise Hämorrhoiden oder dergl. Bei der in der deutschen Offenlegungsschrift 14 91 151 beschriebenen Ausführungsform ist ein
Kern aus Zellstoffwatte vorhanden, der von einer durchlässigen Hülle aus widerstandsfähigerem Material umgeben
ist. Als widerstandsfähigeres Material ist dort "Papiertuch" genannt, ohne daß angegeben wäre, was genau darunter zu verstehen ist. Der vorbekannte Tampon soll etwa
ovalen Querschnitt aufweisen und er besteht über seinen
gesamten Durchmesser betrachtet aus Watte sowie der erwähnten etwas festeren Umhüllung.

Anal-Tampons dieser Art sind im ungebrauchten trockenen
Zustand angenehm weich und nachgiebig, so daß sie ihre
Aufgabe gut erfüllen und auch bei Patienten, die an
schmerzhaften Anal-Erkrankungen leiden, keine Beschwerden

- 3 -

Vereinigte Papierwerke  Schoppershofstr. 80
Schickedanz & Co.  8500 Nürnberg

1 2. Dez. 1985
HP/2 - B 73 D

- 2 -

hervorrufen. Sobald derartige Tampons aber naß werden,
klumpt die Watte zusammen und wird dabei mehr oder weniger hart. Der anfangs angenehm lindernde Tampon wird nun
zur Belastung und muß alsbald ausgetauscht werden.

Der Erfindung liegt die Aufgabe zugrunde, einen Anal-Tampon anzugeben, der auch bei Benässung nicht klumpt und
der seine Elastizität und Formstabilität beibehält. Zur
Lösung dieser Aufgabe wird vorgeschlagen, daß im Innern
des Saugkörpers ein Luftkissen angeordnet ist. Das Luftkissen hat vorzugsweise die Form eines allseits geschlossenen mit Luft oder einem anderen Gas gefüllten Folienschlauches. Der Folienschlauch kann dabei in mehrere Kammern unterteilt sein, eine Maßnahme, die aber nicht
zwangsläufig ist. Bei einer anderen Form des Luftkissens
kann dieses aus einer spiralig aufgewickelten Luftpolsterfolie bestehen. Bei Versuchen hat sich gezeigt, daß
das Luftkissen die Tendenz des Saugstoffes, bei Benässung
zusammenzufallen, fast vollständig auffängt, und daß ein
so aufgebauter Anal-Tampon seine weichen und elastischen
Eigenschaften während der gesamten Gebrauchsdauer beibehält. Die Querschnittsform des Tampons kann oval oder
rund sein.

Die Anordnung des Luftkissens im Innern des Saugkörpers
erhöht die Elastizität und auch die Formstabilität. Um
letzteres zu demonstrieren, wurden Anal-Tampons mit und
ohne Luftkissen hergestellt, welche jeweils eine Länge

- 4 -

Vereinigte Papierwerke
Schickedanz & Co.

Schoppershofstr. 80
8500 Nürnberg

HP/3 - B 73 D    1 2. Dez. 1985

- 3 -

von 9 cm aufwiesen und eine ovale Querschnittform besaßen. Der kleine Durchmesser des Ovals betrug dabei 1,5 cm
und der große Durchmesser 3 cm. Gemessen wurde die prozentuale Differenz des kleinen Durchmessers im trockenen
und im nassen Zustand sowohl bei starker als auch bei
schwacher Belastung. Die nachfolgende Tabelle zeigt die
Ergebnisse der Messung.

Tabelle

| | | |
|---|---|---|
| Tampon ohne Luftkissen | schwache Belastung | 37 % Diff. |
| Tampon ohne Luftkissen | starke Belastung | 37 % " |
| Tampon mit Luftkissen | schwache Belastung | 7 % Diff. |
| Tampon mit Luftkissen | starke Belastung | 6,2 % " |

Die Ergebnisse demonstrieren, daß Tampons mit Luftkissen
auch bei Befeuchtung ihre Formstabilität und damit ihre
Spreiz- und Belüftungswirkung bei Gebrauch behalten.

Die Erfindung wird nachfolgend anhand der Zeichnung näher
erläutert. Es stellen dar:

Fig. 1 eine perspektivische Ansicht einer Ausführungs-
       form eines Anal-Tampons;

Vereinigte Papierwerke          Schoppershofstr. 80
Schickedanz & Co.               8500 Nürnberg

                                HP/4 - B 73 D    12. Dez. 1985

- 4 -

Fig. 2 eine perspektivische Ansicht der noch nicht zu-
       sammengerollten Ausgangsstoffe für die Herstel-
       lung des Tampons;
Fig. 3 eine vergrößerte perspektivische Ansicht einer
       Ausführungsform eines Luftkissens.

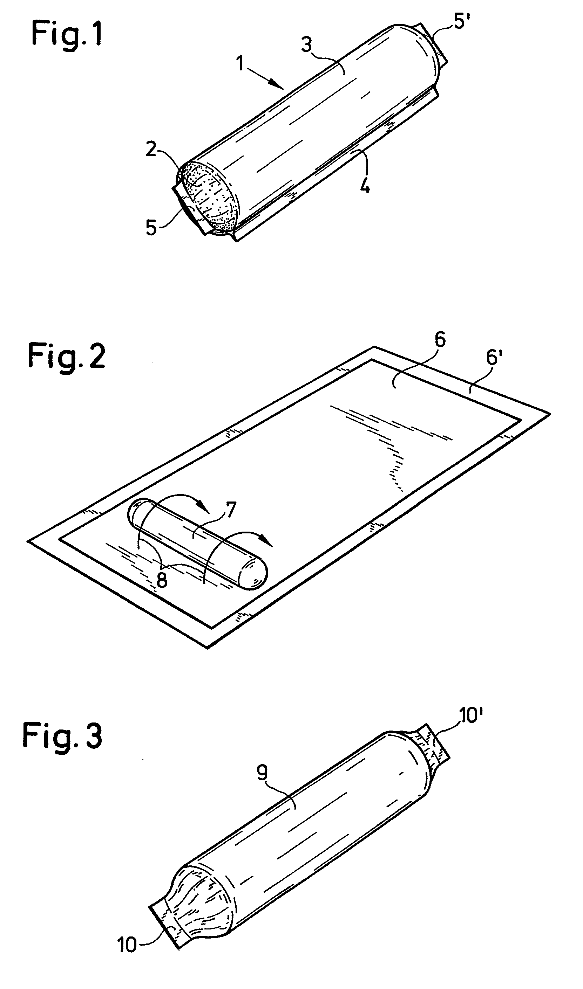

Der in Fig. 1 dargestellte Anal-Tampon ist als Ganzes
mit 1 bezeichnet. Er hat die Form einer langgestreckten
Rolle mit ovalem Querschnitt. Der Tampon besteht aus
einem Saugkörper 2, der im dargestellten Ausführungsbeispiel aus einem ungepreßten Wickel aus Textilwatte besteht. Anstelle reiner Textilwatte kann auch eine Lage
aus Textilwatte und eine Lage aus Zellstoff-Flocken
verwendet werden und es können gegebenenfalls auch die
erwähnten Ausgangsstoffe mit Quellstoffen vermischt
werden. Um die Fasern des Saugkörpers zusammenzuhalten,
ist der Wickel außen mit einer flüssigkeitsdurchlässigen
Hülle 3 umgeben, die im Ausführungsbeispiel aus Vliesstoff besteht. Die Hülle ist an einer Seite durch eine
längsaxiale Schweißnaht 4 und an den beiden Stirnseiten
durch kurze Schweißnähte 5 und 5' verschlossen.

Zur Herstellung eines Anal-Tampons nach Fig. 1 kann von
einem oder mehreren Watteabschnitten 6; 6' ausgegangen
werden. Bei dem in Fig. 2 dargestellten Beispiel sind
zwei Watteabschnitte geringfügig unterschiedlicher Größe
aufeinandergelegt. Auf ein Ende des so entstehenden Wat-

- 6 -

Vereinigte Papierwerke
Schickedanz & Co.

Schoppershofstr. 80
8500 Nürnberg

.1 2. Dez. 1965

HP/5 - B 73 D

- 5 -

testapels wurde ein Luftkissen 7 aufgelegt und die Watteabschnitte sodann in Richtung der beiden Pfeile 8 spiralförmig um das Luftkissen gewickelt. Der so entstandene Rohling wurde alsdann mit einer in Fig. 2 nicht dargestellten Vliesstoffhülle umwickelt und diese verschlossen.

Das Luftkissen 7 hat die Form eines allseits geschlossenen und mit Luft (oder einem anderen Gas, beispielsweise Stickstoff oder Kohlensäure) gefüllten Folienschlauches 9, der beispielsweise in Fig. 3 dargestellt ist. Zur Herstellung kommen Folien aus Polyäthylen oder wegen der Dichtigkeit besser aus Polyvinylchlorid oder PVC-Polystyrol-Zweischichtenfolien in Betracht. Die Folien sind in bekannter Weise zu einem Schlauch geformt, der vorzugsweise ebenfalls ovalen Durchmesser aufweist. Der kleine Durchmesser des Ovals kann dabei etwa 0,8 cm und der große Durchmesser etwa 1,6 cm betragen. Der Schlauch ist an seinen beiden Enden gasdicht zusammengeschweißt; die entstehenden Schweißstellen sind in Fig. 3 mit 10 und 10' bezeichnet.

In Abweichung der in Fig. 3 gewählten Darstellung kann das Luftkissen auch in mehrere Kammern unterteilt sein. Desgleichen wird vorgeschlagen, ein derartiges Luftkissen aus einer spiralig aufgewickelten Luftpolsterfolie herzustellen.

0229907

Vereinigte Papierwerke
Schickedanz &.Co.

Schoppershofstr. 80
8500.Nürnberg

HP/7 - B 73 D    1 2. Dez. 1985

## Bezugszeichenliste

| | | |
|---|---|---|
| 1 | = | Anal-Tampon |
| 2 | = | Saugkörper |
| 3 | = | Hülle |
| 4 | = | Schweißnaht |
| 5; 5' | = | Schweißnähte |
| 6; 6' | = | Watte-Abschnitte |
| 7 | = | Luftkissen |
| 8 | = | Pfeile |
| 9 | = | Folienschlauch |
| 10; 10' | = | Schweißstellen |

- 1 -

Vereinigte Papierwerke
Schickedanz & Co.

Schoppershofstr. 80
8500 Nürnberg

HP/6 - B 73 D 1 2. Dez. 1985

## Patentansprüche

1. Anal-Tampon mit einem Saugkörper aus Textilwatte,
   Zellstoff-Flocken oder dergl., der von einer flüssigkeitsdurchlässigen Hülle umgeben ist,
   dadurch gekennzeichnet,
   daß im Innern des Saugkörpers (2) ein Luftkissen (7)
   angeordnet ist.

2. Anal-Tampon nach Anspruch 1,
   dadurch gekennzeichnet,
   daß das Luftkissen (7) ein allseits geschlossener mit
   Luft oder einem anderen Gas gefüllter Folienschlauch
   (9) ist.

3. Anal-Tampon nach einem der Ansprüche 1 oder 2,
   dadurch gekennzeichnet,
   daß das Luftkissen (7) in mehrere Kammern unterteilt
   ist.

4. Anal-Tampon nach einem der Ansprüche 1 - 3,
   dadurch gekennzeichnet,
   daß das Luftkissen (7) aus einer spiralig aufgewickelten Luftpolster-Folie besteht.

0229907

1/1

Fig.1

Fig.2

Fig.3

**Europäisches Patentamt**

**EUROPÄISCHER RECHERCHENBERICHT**

| | EINSCHLÄGIGE DOKUMENTE | | EP 86115488.8 |
|---|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | DE - A - 1 491 151 (FOLBERTH)<br>---- | | A 61 F 13/20 |

RECHERCHIERTE SACHGEBIETE (Int. Cl. 4)

A 61 F

Der vorliegende Recherchenbericht wurde für alle Patentansprüche erstellt.

| Recherchenort | Abschlußdatum der Recherche | Prüfer |
|---|---|---|
| WIEN | 23-03-1987 | FARNIOK |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82